# EUROPEAN PATENT APPLICATION

(11) **EP 4 660 254 A1**
(43) Date of publication of application: **10.12.2025**
(21) Application number: 24920729.1
(22) Date of filing: 15.07.2024
(51) Int. Cl.: C09B 23/08, C09K 11/06, A61K 49/00, C07K 5/083, C07K 7/06, C07K 7/08

(54) **HEPTAMETHINE CYANINE NEAR-INFRARED FLUORESCENT DYE, PREPARATION METHOD THEREFOR, AND USE THEREOF**

(30) Priority: 16.04.2024 CN 202410451606
(71) Applicant: Nanjing Nuoyuan Medical Devices Co., Ltd, Nanjing, Jiangsu 210000 (CN)
(72) Inventor: LI, Changsheng, Nanjing, Jiangsu 210000 (CN); CAI, Huiming, Nanjing, Jiangsu 210000 (CN); ZHANG, Huidan, Nanjing, Jiangsu 210000 (CN)
(74) Representative: Cleanthous, Marinos
(86) International application number: PCT/CN2024/105429
(87) International publication number: WO 2025/218036

(57) **Abstract**

A heptamethine cyanine near-infrared fluorescent dye as well as a preparation method and use thereof are provided, and relate to the technical field of organic fluorescent molecules. The heptamethine cyanine near-infrared fluorescent dye has a structure represented by formula I. The heptamethine cyanine near-infrared fluorescent dye provided in the present disclosure has better in-vivo metabolic characteristics, no accumulation in normal tissues such as liver and long tumor tissue developing window time, is more suitable for precise diagnosis of tumors, possesses a certain clinical application prospect, and can be applied to fluorography of clinical tumors and the like.

## Description

### TECHNICAL FIELD

The present disclosure relates to the technical field of organic fluorescent molecules, particularly to a heptamethine cyanine near-infrared fluorescent dye as well as a preparation method and use thereof.

### BACKGROUND

Cancers severely threaten human's life health. With the deepening of population aging and changes in people's lifestyles, the challenges brought by cancers are becoming increasingly severe. At present, treatment means for cancers mainly include chemotherapy, radiation therapy, ablation, surgical resection, etc. As for solid tumors, early screening, early diagnosis and early surgery can effectively improve the curative rate of diseases and thus prolong the lifetime of patients.

With the emergence of various advanced medical equipment, such as computed tomography (CT), magnetic resonance imaging (MRI), positron-emission tomography (PET), a near-infrared fluorescence imaging system and ultrasound (US), the early detection rate of tumors is greatly improved, thereby providing important reference for precise diagnosis of tumors. Compared with CT and MRI, an optical molecular imaging surgical navigation technology has the characteristics of real time, no invasion and high resolution, and therefore provides a new imageology aided means for precise surgery of tumors. Near-infrared fluorescence imaging diagnosis has the advantages in tumor diagnosis (early, mid and advanced), intraoperative navigation, prognosis, recurrence monitoring diagnosis, etc., which conducts specific detection through a near-infrared fluorescence probe (650-1000 nm), can provide high-resolution tissue and organ images, has the advantages of small biotoxicity and low autofluorescence, and is beneficial for reducing background interference to the greatest extent.

Indocyanine green is a near-infrared fluorescent dye approved by FDA, which is prone to binding to a plasma protein after intravenous injection, is metabolized into bile via liver, and can be retained in tumor tissues via an enhanced permeability and retention (EPR) effect. Indocyanine green has high safety and a broad-spectrum tumor targeting effect (all solid tumors), but it has a relatively weak tumor targeting ability, with a clinical human dose of ≤ 2.0 mg/kg, and therefore its application range is limited. Hence, it is clinical significance for developing a targeted contrast agent with a broad spectrum, higher fluorescence efficiency and stronger tumor targeting ability.

CN111196896A discloses a water-soluble heptamethine cyanine near-infrared fluorescent dye having tumor targeting and use thereof. After such the near-infrared fluorescent dye having a structural formula I is widely distributed in tissues of the whole body along with hemoglobins, its metabolism in normal tissues is faster than that in tumor tissues, and therefore a large number of near-infrared fluorescent dye can be retained in tumor tissues, thereby playing a role in live diagnosis. After tumor targeting ability verification, it is found that compared with ICG, NIR-04 has a stronger tumor targeting ability, and tumor retention time of over 48 h; however, NIR-04 has poor long-term accumulation in liver.

Accordingly, it is urgent to develop a near-infrared fluorescent dye having a strong tumor targeting ability and long tumor imaging time.

In view of this, the present disclosure is proposed.

### SUMMARY

The first objective of the present disclosure is to provide a heptamethine cyanine near-infrared fluorescent dye. The heptamethine cyanine near-infrared fluorescent dye is modified using NIR-04 as a mother nucleus, thereby effectively improving poor long-time accumulation of NIR-04 in liver while retaining the tumor targeting ability; that is to say, the heptamethine cyanine near-infrared fluorescent dye of the present disclosure has the advantages of strong tumor targeting ability and long tumor imaging time.

The second objective of the present disclosure is to provide a preparation method of a heptamethine cyanine near-infrared fluorescent dye. By using the preparation method, a series of heptamethine cyanine near-infrared fluorescent dyes are prepared by further modifying NIR-04 through amide condensation under the action of a condensing agent, deprotection of a triphenylmethyl protecting group with triphenylacetic acid, nucleophilic substitution, further deprotection and other reactions.

The third objective of the present disclosure is to provide use of the heptamethine cyanine near-infrared fluorescent dye in preparation of a fluorescent contrast agent.

The fourth objective of the present disclosure is to provide use of the heptamethine cyanine near-infrared fluorescent dye in preparation of a drug for tumor diagnosis; especially use in preparation of a drug for liver cancer or colorectal cancer diagnosis.

In order to achieve the above objectives of the present disclosure, the following technical solution is adopted:
In a first aspect, the present disclosure provides a heptamethine cyanine near-infrared fluorescent dye having a structure represented by formula I: 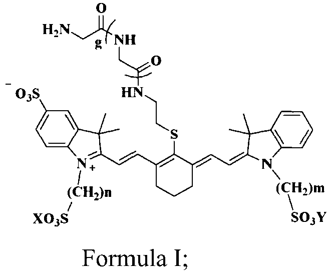 wherein, X or Y is each independently a hydrogen ion or a salifiable positive-valence ion; m or n is each independently 3 or 4; and g is an integer from 0 to 20, which for example can be 0, 1, 2, 3, 4, 5, 6, 7, 8, 9, 10, 11, 12, 13, 14, 15, 16, 17, 18, 19, 20, etc.

In the present disclosure, the heptamethine cyanine near-infrared fluorescent dye is modified by using NIR-04 as a mother nucleus to obtain a passively targeted broad-spectrum fluorescent contrast agent having good water solubility, no liver accumulation and long tumor site retention time, thereby being applied to clinical tumor diagnosis and intraoperative navigation.

Preferably, the salifiable positive-valence ion comprises a positive-valence alkali metal ion and/or NH⁺₄.

Preferably, the positive-valence alkali metal ion is Na⁺ and/or K⁺.

In a second aspect, the present disclosure provides a preparation method of the heptamethine cyanine near-infrared fluorescent dye as described in the first aspect, comprising the following steps:
(1) mixing ((9H-fluoren-9-yl) methoxy) carbonyl triglycine, 2-(triphenylmethylsulfydryl) ethylamine, a condensing agent, an alkali and a reaction solvent, performing a condensation reaction on the above mixed solution and then posttreating the obtained condensation product, so as to obtain an intermediate a;
(2) mixing the intermediate a, trifuloroacetic acid, triisopropylsilane and water for reaction, and then posttreating the obtained reaction product to obtain an intermediate b;
(3) mixing a dye molecule, the intermediate b, the alkali and the reaction solvent for reaction, and then posttreating the obtained reaction product to obtain an intermediate c;
   the dye molecule having a structure represented by formula II: 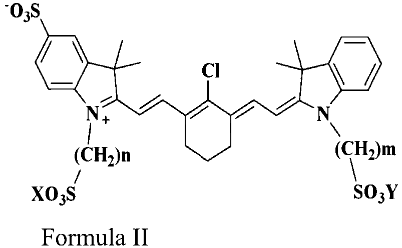 wherein, X or Y is each independently a hydrogen ion or a salifiable positive-valence ion; m or n is each independently 3 or 4; and
(4) mixing the intermediate c, secondary amine and the reaction solvent for reaction, and then posttreating the obtained reaction product to obtain the heptamethine cyanine near-infrared fluorescent dye.

Preferably, in step (1), a molar ratio of the ((9H-fluoren-9-yl) methoxy) carbonyl triglycine to the 2-(triphenylmethylsulfydryl) ethylamine to the condensing agent to the alkali is 1: (0.8-1.2): (1.5-3): (2-4);
wherein, "0.8-1.2" for example can be 0.8, 0.9, 1, 1.1, 1.2, etc.;
wherein, "1.5-3" for example can be 1.5, 1.8, 2, 2.2, 2.5, 3, etc.; and
wherein, "2-4" for example can be 2, 2.5, 3, 3.5, 4, etc.

Preferably, in step (1), a mass ratio of the reaction solvent to the ((9H-fluoren-9-yl) methoxy) carbonyl triglycine is (5-20): 1, which for example can be 5:1, 6:1, 8:1, 10:1, 12:1, 14:1, 16:1, 18:1, 20:1, etc.

Preferably, in step (1), the condensing agent is selected from a combination of any one or at least two of 2-(7-azabenotriazole)-N,N,N,N,-tetramethylurea hexafluorophosphate (HATU), O-(benzotriazol-1-yl)-N,N,N',N'-tetramethyluronium (HBTU), O-(6-chloro-1-hydrocibenzotriazol-1-yl)-1,1,3,3-tetramethyluronium hexafluorophosphate (HCTU), 1-hydroxy-7-oxo-6-azo-3,4-dihydroquinoline-2,5-dione (HOAT) or 1-hydroxybenzotriazole (HOBT).

Preferably, in step (1), the alkali is selected from triethylamine and/or diisopropylethylamine.

Preferably, in step (1), the reaction solvent is a polar solvent.

Preferably, in step (1), the polar solvent is selected from a combination of any one or at least two of dimethylformamide, dimethyl sulfoxide or N-methylpyrrolidone.

Preferably, in step (1), the temperature of the condensation reaction is 10-40°C, which for example can be 10°C, 15°C, 20°C, 25°C, 30°C, 35°C, 40°C, etc.; and the time of the condensation reaction is 0.5-2 h, which for example can be 0.5 h, 0.6 h, 0.8 h, 1 h, 1.2 h, 1.4 h 1.6 h 1.8 h, 2 h, etc.

Preferably, in step (1), the posttreating specifically comprises the following steps: adding water into a reaction solution obtained after condensation reaction in step (1) to form a suspension; and extracting the suspension with an organic solvent, collecting an organic phase, and then drying and concentrating the collected organic phase to obtain the intermediate a.

Preferably, in step (1), the organic solvent used for the extraction is ethyl acetate;

Preferably, in step (1), a volume ratio of the reaction solution to the water to the organic solvent is 1: (0.8-1.2): (1-5);
wherein, "0.8-1.2" for example can be 0.8, 0.9, 1, 1.1, 1.2, etc.; and
wherein, "1-5" for example can be 1, 1.5, 2, 2.5, 3, 3.5, 4, 4.5, 5, etc.

Preferably, in step (2), a volume ratio of the trifluoroacetic acid to the triisopropylsilane to the water is (94-96): (2-3): (2-3);
wherein, "94-96" for example can be 94, 94.5, 95, 95.5, 96, etc.; and
wherein, "2-3" for example can be 2, 2.2, 2.4, 2.6, 2.8, 3, etc.

Preferably, in step (2), a ratio of a total volume of a mixed solution of the trifluoroacetic acid, the triisopropylsilane and the water to a volume of the organic solvent for extraction is 1: (0.8-1.2), which for example can be 1: 0.8, 1: 0.9, 1: 1, 1: 1.1, 1: 1.2, etc.

Preferably, in step (2), the temperature of the reaction is 10-40°C, which for example can be 10°C, 15°C, 20°C, 25°C, 30°C, 35°C, 40°C, etc.; and the time of the reaction is 0.5-2 h, which for example can be 0.5 h, 0.6 h, 0.8 h, 1 h, 1.2 h, 1.4 h, 1.6 h, 1.8 h, 2 h, etc..

Preferably, in step (2), the posttreating specifically comprises the following steps: the pH of a reaction solution obtained after reaction in step (2) is adjusted to be neutral, and then performing filtration, concentration and column chromatography on the solution after pH adjustment to obtain the intermediate b.

Preferably, in step (2), a reagent for adjusting the pH is a sodium carbonate solution.

Preferably, in step (3), a molar ratio of the dye molecule to the intermediate b to the alkali is 1: (0.8-1.2): (1.5-4);
wherein, "0.8-1.2" for example can be 0.8, 0.9, 1, 1.1, 1.2, etc.; and
wherein, "1.5-4" for example can be 1.5, 2, 2.5, 3, 3.5, 4, etc.

Preferably, in step (3), a mass ratio of the reaction solvent to the intermediate b is (5-20):1, which for example can be 5:1, 6:1, 8:1, 10:1, 12:1, 14:1, 16:1, 18:1, 20:1, etc.

Preferably, in step (3), the alkali is selected from triethylamine and/or diisopropylethylamine.

Preferably, in step (3), the reaction solvent is a polar solvent.

Preferably, the polar solvent is selected from a combination of any one or at least two of dimethylformamide, dimethyl sulfoxide or N-methylpyrrolidone.

Preferably, in step (3), the temperature of the reaction is 10-40°C, which for example can be 10°C, 15°C, 20°C, 25°C, 30°C, 35°C, 40°C, etc.; and the time of the reaction is 0.5-2 h, which for example can be 0.5 h, 0.6 h, 0 .8 h, 1 h, 1.2 h, 1.4 h, 1.6 h, 1.8 h, 2 h, etc.

Preferably, in step (3), the posttreating specifically comprises the following steps: adding an organic solvent into the reaction solution obtained after reaction in step (3), and centrifuging and filtering the above mixed solution to obtain the intermediate c.

Preferably, in step (3), the organic solvent is ethyl acetate and/or methyl tert-butyl ether.

Preferably, in step (4), a molar ratio of the intermediate c to the secondary amine is 1: (1-2), which for example can be 1:1, 1: 1.1, 1:1.2, 1:1.3, 1:1.4, 1:1.5, 1:1.6, 1:1.7, 1:1.8, 1:1.9, 1:2, etc.

Preferably, in step (4), a mass ratio of the reaction solvent to the intermediate c is (5-20): 1, which for example can be 5:1, 6:1, 8:1, 10:1, 12:1, 14:1, 16:1, 18:1, 20:1, etc.

Preferably, in step (4), the secondary amine is selected from a combination of any one or at least two of piperidine, morpholine or diethylamine.

Preferably, in step (4), the reaction solvent is a polar solvent.

Preferably, in step (4), the polar solvent is selected from dimethylformamide, dimethyl sulfoxide or N-methylpyrrolidone.

Preferably, in step (4), the temperature of the reaction is 10-40°C, which for example can be 10°C, 15°C, 20°C, 25°C, 30°C, 35°C, 40°C, etc.; and the time of the reaction is 2-6 h, which for example can be 2 h, 2.5 h, 3 h, 3 .5 h, 4 h, 4.5 h, 5 h, 5.5 h, 6 h, etc.

Preferably, in step (4), the posttreating specifically comprises the following steps: adding an organic solvent into the reaction solution obtained after reaction in step (4) and filtering the above mixed solution to obtain a crude product, and then purifying the crude product via liquid chromatography to obtain the heptamethine cyanine near-infrared fluorescent dye.

Preferably, in step (4), the organic solvent is ethyl acetate and/or methyl tert-butyl ether.

More specifically, the heptamethine cyanine near-infrared fluorescent dye obtained in step (4) has the structure represented by formula I, and g is 2.

As an optional technical solution of the present disclosure, the preparation method of the heptamethine cyanine near-infrared fluorescent dye further comprises the following steps:
(5) mixing the heptamethine cyanine near-infrared fluorescent dye obtained in step (4), a raw material 2, the condensing agent, the alkali and the reaction solvent, performing a condensation reaction on the above materials and then posttreating the condensation product to obtain the intermediate e; wherein the raw material 2 is selected from ((9H-fluoren-9-yl) methoxy) carbonyl triglycine or BOC-glycine 3-COOH; and
(6) performing a deprotection reaction on the intermediate e and then posttreating the obtained reaction product to obtain the heptamethine cyanine near-infrared fluorescent dye.

More specifically, the heptamethine cyanine near-infrared fluorescent dye obtained in step (6) has the structure represented by formula I, and g is 5.

Preferably, in step (5), a molar ratio of the raw material 2 to the heptamethine cyanine near-infrared fluorescent dye obtained in step (4) to the condensing agent to the alkali is 1: (0.8-1.2): (1.5-3): (2-4);
wherein, "0.8-1.2" for example can be 0.8, 0.9, 1, 1.1, 1.2, etc.;
wherein, "1.5-3" for example can be 1.5, 1.8, 2, 2.2, 2.5, 3, etc.;
wherein, "2-4" for example can be 2, 2.5, 3, 3.5, 4, etc.

Preferably, in step (5), a mass ratio of the reaction solvent to the raw material 2 is (5-20):1, which for example can be 5:1, 6:1, 8:1, 10:1, 12:1, 14:1, 16:1, 18:1, 20:1, etc.

Preferably, in step (5), the condensing agent is selected from a combination of any one or at least two of HATU, HBTU, HCTU, HOAT or HOBT.

Preferably, in step (5), the alkali is selected from triethylamine and/or diisopropylethylamine.

Preferably, in step (5), the reaction solvent is a polar solvent.

Preferably, in step (5), the polar solvent is selected from a combination of any one or at least two of dimethylformamide, dimethyl sulfoxide or N-methylpyrrolidone.

Preferably, in step (5), the temperature of the condensation reaction is 10-40°C, which for example can be 10°C, 15°C, 20°C, 25°C, 30°C, 35°C, 40°C, etc.; and the time of the condensation reaction is 0.5-2 h, which for example can be 0.5 h, 0.6 h, 0.8 h, 1 h, 1.2 h, 1.4 h 1.6 h 1.8 h, 2 h, etc.

Preferably, in step (5), the posttreating specifically comprises the following steps: adding an organic solvent into the reaction solution obtained after condensation reaction in step (5) and then filtering and drying the above mixed solution to obtain the intermediate e.

Preferably, in step (5), the organic solvent added into the reaction solution is ethyl acetate and/or methyl tert-butyl ether.

Preferably, in step (6), the reagent for deprotection is secondary amine and/or trifluoroacetic acid.

Preferably, in step (b), the secondary amine used for deprotection is selected from a combination of any one or at least two of piperidine, morpholine or diethylamine.

Preferably, step (5) and step (6) are repeated until the heptamethine cyanine near-infrared fluorescent dye having the structure represented by formula I, in which g is more than 6, is obtained.

In a third aspect, the present disclosure provides use of the heptamethine cyanine near-infrared fluorescent dye as described in the first aspect in preparation of a fluorescent contrast agent.

In a fourth aspect, the present disclosure provides use of the heptamethine cyanine near-infrared fluorescent dye as described in the first aspect in preparation of a drug for tumor diagnosis.

Preferably, the tumor is liver cancer or colorectal cancer.

Compared with the prior art, the present disclosure has the following beneficial effects:
(1) a heptamethine cyanine near-infrared fluorescent dye is prepared by further modifying NIR-04 through amide condensation, deprotection of a triphenylmethyl protecting group with triphenylacetic acid, nucleophilic substitution, deprotection and other reactions, thereby improving the in-vivo metabolic characteristic of NIR-04, without liver accumulation and other deficiencies, and the fluorescent dye can be used for tumor resection in surgical navigation.
(2) The heptamethine cyanine near-infrared fluorescent dye prepared in the present disclosure has a broad-spectrum passive tumor targeting effect, has stronger tumor targeting ability than ICG at the same dose, possesses the advantages of good water solubility, long tumor retention time, lower dose, no accumulation in normal tissues and the like, not only can be applied to preparation of fluorescent contrast agents or tumor diagnosis drugs, but also has application prospects in the fields of fluorescent guidance of surgical resection of tumors in clinical surgery.

### BRIEF DESCRIPTION OF THE DRAWINGS

For more clearly illustrating specific embodiments of the present disclosure or technical solutions in the prior art, drawings required to be used in specific embodiments or in the prior art will be simply introduced, obviously, the drawings in the following description are some embodiments of the present disclosure. For persons of ordinary skill in the art, other drawings can be obtained according to these drawings without creative efforts.
FIG. 1 is an absorption spectrogram of NY-ICG-04-01 to NY-ICG-04-04 prepared in example 1 to example 4.
FIG. 2 is a fluorescence spectrogram of NY-ICG-04-01 to NY-ICG-04-04 prepared in example 1 to example 4.
FIG. 3 is a distribution imaging result graph of NY-ICG-01 to NY-ICG-03 prepared in example 1 to example 3 and NY-ICG-04 provided in comparative example 1 in each organ of normal BCR mice.
FIG. 4 is an in-vivo imaging result graph of NY-ICG-04-01 to NY-ICG-04-04 prepared in example 1 to example 3 and ICG provided in comparative example 2 in liver cancer HepG2 tumor-bearing mice.
FIG. 5 is an in-vivo imaging result graph of NY-ICG-04-03 prepared in example 3 in colorectal cancer HCT116 tumor-bearing mice.

### DETAILED DESCRIPTION OF THE EMBODIMENTS

Unless defined otherwise, scientific and technical terms used in combination with the present disclosure should have the meaning understood by persons of ordinary skill in the art. The meaning and range of the terms should be clear, however, in any case of potential ambiguity, the definition provided in this article is superior to any dictionary or foreign definition. In the present application, unless stated otherwise, the use of "or" means "and/or". In addition, the use of the term "comprise" and other forms is non-limiting.

It is noted that specific details are demonstrated in the following description so as to fully understand the present disclosure. However, the present disclosure can be implemented in multiple manners different from the manners described here, and those skilled in the art can make similar promotion without obeying the concept of the present disclosure. Therefore, the present disclosure is not limited by specific embodiments disclosed below.

Next, the technical solution of the present disclosure will be clearly and completely described in combination with embodiments, obviously, the described embodiments are only some embodiments of the present disclosure but not all the embodiments. Based on the embodiments of the present disclosure, all other embodiments obtained by persons of ordinary skill in the art without creative efforts belong to the protective scope of the present disclosure.

Next, the present disclosure will be further illustrated through examples. Unless otherwise specified, materials used in examples are prepared according to the existing methods, or directly purchased in the market.

Materials in examples and comparative examples and their sources are as follows:

| Materials | Manufacturers | Article numbers |
|---|---|---|
| 2-(7-azabenotriazole)-N,N,N,N,-tetramethylurea hexafluorophosphate (HATU) | Acon Biotech (Hangzhou) Co., Ltd. | AK001LKC |
| N,N-diisopropylethylamine (DIPEA) | J & K Scientific | Cat NO:203402 |
| ((9H-fluoren-9-yl) methoxy)carbonyl triglycine | Bidepharm | BD39294 |
| BOC-glycine 3-COOH | Nanjing Zhongda Tianqing Glass Instrument Co., Ltd | GLS230320 |
| 2-(triphenylmethylsulfydryl) ethylamine | Bidepharm | BD00776855 |
| Triisopropyl silane (Et₃SiH) | Bidepharm | BD155373 |
| Morpholine | Innochem | A60327 |
| Ethyl acetate | Nanjing Chemical Reagent Co., Ltd | GB/T12589-2007 |
| Ethyl acetate | Aladdin | T103291 |
| DMSO | Nanjing Chemical Reagent Co., Ltd | HG/T 5345-2018 |
| NY-ICG-04 (NIR-04) | Self-made | - |

### Example 1

This example provides a heptamethine cyanine near-infrared fluorescent dye, which is NY-ICG-04-01:

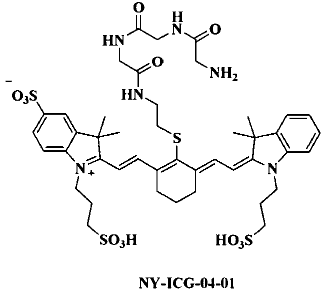

The synthesis route of the heptamethine cyanine near-infrared fluorescent dye NY-ICG-04-01 is as follows:

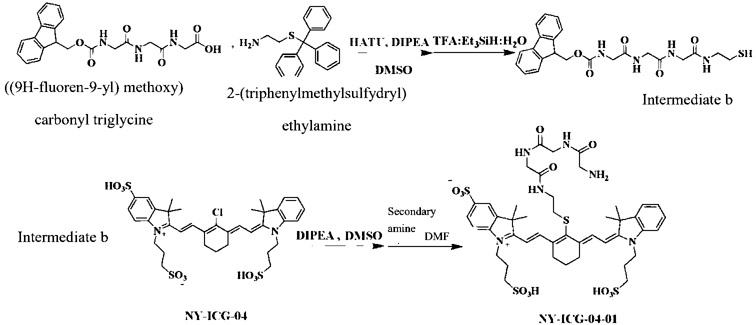

A preparation method of the heptamethine cyanine near-infrared fluorescent dye NY-ICG-04-01 specifically comprises the following steps:
(1) ((9H-fluoren-9-yl) methoxy) carbonyl triglycine (1 g, 1.0 eq), 2-(triphenylmethylsulfydryl) ethylamine (776 mg, 1.0 eq), HATU (1.85 g, 2.0 eq), N,N-diisopropylethylamine (941 mg, 3.0 eq) and DMSO (10 mL, 10 V) were mixed, and then reacted for 1 h at room temperature via thin layer chromatography (TLC) (DCM:MeOH = 50:1) monitoring, 10 mL of water was dropwise added after the reaction was completed, and then the solution after reaction was extracted three times with ethyl acetate (10 mL × 3), washed twice with a saturated salt solution (20 mL × 2), dried over anhydrous sodium sulfate and then concentrated to obtain an intermediate a.
(2) A mixed solution (10 mL ) of trifluoroacetic acid, triisopropyl silane and water was added into the intermediate a, wherein a volume ratio of trifluoroacetic acid to triisopropyl silane to water was 95:2.5:2.5; the above-mentioned materials reacted for 1 h at room temperature via TLC monitoring, the TLC monitoring showed that after the reaction was completed, the pH was adjusted with a sodium carbonate solution to be neutral so as to precipitate out a white solid, and then the precipitated white solid was filtered and then dried, followed by column chromatography, so as to obtain an intermediate b.

Structural characterization was performed via mass spectrum and ¹H NMR. The structure determination result is as follows:
LCMS (ESI): m/z: C₂₃H₂₆N₄O_{S}, [M+H]⁺ calcd for 470 .16; found, 470.8.

¹H NMR (600 MHz, DMSO-d6): δ 8.16 (ddt, J = 17.4, 11.5, 5.8 Hz, 2H) ,8.01-7.84 (m, 3H), 7.73 (dt, J = 13.4, 7.4 Hz, 2H), 7.59 (t, J=6.2 Hz, 1H), 7.45-7.29 (m, 4H), 4.30 (t, J = 6.6 Hz, 1H), 4.26-4.15 (m, 1H), 3.78-3.72 (m, 2H), 3.68 (t, J =6.1 Hz, 3H), 3.34 (s, 7H), 3.26-3.19 (m, 2H), 2.58-2.52 (m, 1H), 1.36-1.17 (m, 1H).

(3) NY-ICG-04 (100 mg, 1.0 eq), the intermediate b (66 mg, 1.1 eq), DIPEA (50 mg, 3.0 eq) and DMSO (1 mL, 10 V) were mixed, and then reacted for 1 h at room temperature via HPLC monitoring, the HPLC monitoring showed that after NY-ICG-04 reaction was completed, ethyl acetate (10 mL) was dropwise added into the reaction solution to precipitate out a green precipitate, and then the green precipitate was centrifuged to obtain an intermediate c.

(4) DMF (1 mL) and morpholine (17 mg, 1.5 eq) were added into the intermediate c, the above materials reacted for 4 h at room temperature via HPLC monitoring, the HPLC monitoring showed that after the reaction was completed, ethyl acetate (10 mL) was dropwise added into the reaction solution to precipitate out a green precipitate, the green precipitate was purified via preparative liquid chromatography to obtain a target fraction solution, and the obtained target fraction solution was then freeze-dried to obtain a near-infrared fluorescent probe NY-ICG-04-01.

Structural characterization was performed via mass spectrum, ¹H NMR and ¹³C NMR. The structure determination result is as follows:
LCMS (ESI): m/z: [M-H]⁻C₄₄H₅₉N₆O₁₂S₄, calcd for 989.3; found, 989.3.

¹H NMR (600 MHz, DMSO-d6): δ 8.79 (d, J = 14.1 Hz, 1H), 8.69 (d, J = 13.9 Hz, 1H), 8.60 (t, J = 5.7 Hz, 1H), 8.23 (t, J = 5.9 Hz, 1H), 8.12 (t, J = 5.7 Hz, 1H), 7.98 (t, J = 5.9 Hz, 3H), 7.81 (d, J = 1.6 Hz, 1H), 7.66-7.61 (m, 1H), 7.54 (d, J = 8.0 Hz, 1H), 7.46-7.37 (m, 2H), 7.28 (t, J = 7.4 Hz, 1H), 6.56 (d, J = 14.3 Hz, 1H), 6.48 (d, J = 14.1 Hz, 1H), 4.41-4.36 (m, 2H), 4.34-4 29 (m, 2H), 3.82 (d, J = 5.7 Hz, 2H), 3.67 (d, J = 5.8 Hz, 2H), 3.61 (q, J = 5.9 Hz, 2H), 3.27 (q, J = 6.7 Hz, 2H), 2.86 (dd, J = 8.8, 6.2 Hz, 2H), 2.69 (d, J = 5.3 Hz, 3H), 2.61 (t, J = 6.8 Hz, 4H), 2.03 (dt, J = 14.9, 7.5 Hz, 3H), 1.81 (p, J = 7.0, 6.5 Hz, 2H), 1.70 (d, J = 14.4 Hz, 12H).

¹³C NMR (151 MHz, DMSO-d6): δ 173.06, 171.82, 169.21, 168.98, 166.74, 159.22, 158.96, 158.71, 158.45, 154.91, 146.08, 145.15, 144.68, 142.90, 142.63, 141.59, 140.75, 134.31, 133.75, 129.09, 126.58, 125.56, 122.96, 120.34, 116.47, 114.56, 112.65, 112.04, 110.49, 102.82, 101.93, 49.46, 49.01, 48.31, 43.33, 43.14, 42.33, 3.37, 27.91, 27.84, 26.35, 26.27, 23.95, 23.75, 21.13.

### Example 2

This example provides a heptamethine cyanine near-infrared fluorescent dye, which is NY-ICG-04-02:

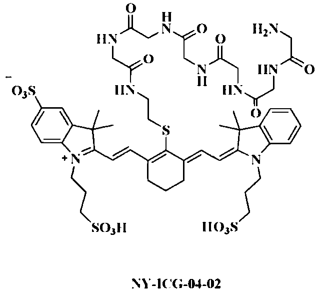

The synthesis route of the heptamethine cyanine near-infrared fluorescent dye NY-ICG-04-02 is as follows:

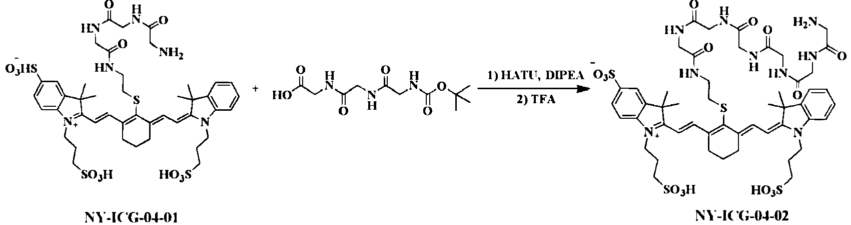

A preparation method of the heptamethine cyanine near-infrared fluorescent dye NY-ICG-04-02 specifically comprises the following steps:
(5) NY-ICG-04-01 (110 mg, 1.0 eq), BOC-glycine 3-COOH (65 mg, 2.0 eq), HATU (127 mg, 3.0 eq), DIPEA (72 mg, 5.0 eq) and DMSO (1.1 mL) were mixed, and then reacted for 1 h at room temperature via HPLC monitoring, after the NY-ICG-04-01 reaction was completed, ethyl acetate (11 mL) was dropwise added into the reaction solution to precipitate out a green flocculent material, and then the green flocculent material was filtered and dried to obtain an intermediate e.

(6) TFA (100 µL) was added into the intermediate e to react for 15 min at room temperature, TFA was removed from the reaction product, and then NY-ICG-04-02 was obtained by purification via preparative liquid chromatography.

Structural characterization was performed via mass spectrum, ¹H NMR and ¹³C NMR. The structure determination result is as follows:
LCMS (ESI): m/z: [M]+ calcd for C₅₀H₆₈NO₁₅S₄, 1163.4; found, 1163.

¹H NMR (600 MHz, DMSO-d6): δ 8.80 (d, J = 14.1 Hz, 1H), 8.73-8.61 (m, 2H), 8.31 (t, J = 5.9 Hz, 1H), 8.16 (t, J = 5.8 Hz, 1H), 8.08 (dt, J = 15.9, 5.9 Hz, 2H), 8.01 (q, J = 6.3 Hz, 4H), 7.78 (s, 1H), 7.66-7.61 (m, 2H), 7.54 (d, J = 8.0 Hz, 1H), 7.43 (t, J = 8.3 Hz, 1H), 7.38 (d, J = 8.4 Hz, 1H), 7.29 (t, J = 7.4 Hz, 1H), 6.56 (d, J =14.3 Hz, 1H), 6.47 (d, J = 14.0 Hz, 1H), 5.57 (s, 1H), 5.52 (s, 1H), 4.39 (t, J = 7.7 Hz, 2H), 4.34-4.28 (m, 2H), 3.84 (d, J = 5.7 Hz, 2H), 3.77-3.68 (m, 6H), 3.63 (q, J = 6.0 Hz, 4H), 3.25 (dt, J= 8.7, 5.2 Hz, 2H), 2.85 (dd, J = 8.8, 6.5 Hz, 2H), 2.69 (d, J = 5.3 Hz, 4H), 2.62 (td, J = 6.8, 3.7 Hz, 4H), 2.04 (dq, J = 14.5, 7.0 Hz, 4H), 1.81 (p, J =6.4 Hz, 2H), 1.70 (d, J = 13.6 Hz, 12H).

¹³C NMR (151 MHz, DMSO-d6): δ 173.15, 171.71, 169.74, 169.54, 169.2, 166.90, 158.96, 158.70, 154.83, 146.14, 145.08, 144.58, 142.92, 142.61, 141.62, 140.69, 134.43, 133.73, 129.09, 126.58, 125.60, 122.96, 120.30, 116.49, 114.57, 112.06, 110.46, 102.89, 101.85, 49.49, 48.99, 48.29, 43.35, 43.13, 42.53, 36.15, 27.90, 27.83, 26.35, 23.94, 23.70, 21.12.

### Example 3

This example provides a heptamethine cyanine near-infrared fluorescent dye, which is NY-ICG-04-03:

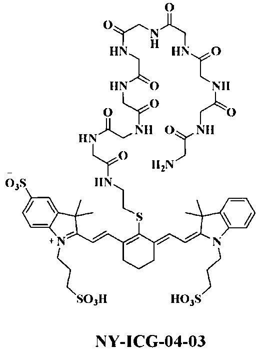

With reference to example 2, NY-ICG-04-03 was obtained by purification via preparative liquid chromatography.

Structural characterization was performed via mass spectrum, ¹H NMR and ¹³C NMR. The structure determination result is as follows:
LCMS (ESI): m/z: [M-2^{]2-}C₅₆H₇₇N₁₂O₁₈S₄, calcd for 665.2; found, 664.9.

¹H NMR (600 MHz, DMSO-d6): δ 8.80 (d, J = 14.1 Hz, 1H), 8.69 (d, J = 13.8 Hz, 1H), 8.65 (t, J = 5.8 Hz, 1H), 8.31 (t, J = 5.8 Hz, 1H), 8.20-8.12 (m, 4H), 8.07 (dt, J = 12.0, 5.8 Hz, 2H), 8.03-7.95 (m, 4H), 7.78 (s, 1H), 7.64 (td, J = 8.2, 1.3 Hz, 2H), 7.54 (d, J = 8.1 Hz, 1H), 7.45-7.41 (m, 1H), 7.38 (d, J = 8.3 Hz, 1H), 7.29 (t, J = 7.4 Hz, 1H), 6.56 (d, J = 14.3 Hz, 1H), 6.46 (d, J = 13.9 Hz, 1H), 5.55 (s, 1H), 5.33 (s, 1H), 4.39 (t, J = 7.7 Hz, 2H), 4.30 (d, J = 7.7 Hz, 2H), 3.85 (d, J = 5.7 Hz, 2H), 3.78-3.68 (m, 12H), 3.64 (t, J = 5.7 Hz, 4H), 3.25 (dt, J = 10.5, 5.8 Hz, 2H), 2.87-2 .82 (m, 2H), 2.69 (q, J = 5.4 Hz, 4H), 2.63 (q, J = 6.4 Hz, 4H), 2.04 (dp, J = 14.7, 7.3 Hz, 4H), 1.81 (p, J = 6.1 Hz, 2H), 1.70 (d, J = 14.5 Hz, 12H).

¹³C NMR (151 MHz, DMSO-d6): δ 173.21, 171.67, 169.83, 169.56, 169.28,166 .92, 158.96, 158.71, 154.86, 146.19, 144.99, 144.54, 142.96, 142.60, 141.64, 140.70, 134.45, 133.71, 129.09, 126.61, 125.62, 122.97, 120.30, 116 .50, 114.59, 112.07, 110.46, 102.92, 101.82, 49.50, 48.99, 48.29, 43.36, 43.12, 42.54, 42.37, 36.16, 27.89, 27.82, 26.35, 26.26, 23.93, 23.68, 21.11.

### Example 4

This example provides a heptamethine cyanine near-infrared fluorescent dye, which is NY-ICG-04-04:

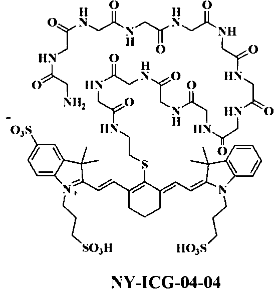

With reference to example 2, NY-ICG-04-04 was obtained by purification via preparative liquid chromatography.

Structural characterization was performed via mass spectrum, ¹H NMR and ¹³C NMR. The structure determination result is as follows:
LCMS (ESI): m/z: [M-2]²⁻C₅₆H₇₇N₁₂O₁₈S₄, calcd for 750.7; found, 751.1

¹H NMR (600 MHz, DMSO-d6): δ 8.80 (d, J = 14.1 Hz, 1H), 8.31 (t, J =5.6 Hz, 1H), 8.16 (dq, J = 9.6, 5.3 Hz, 6H), 8.06 (dd, J = 9.7, 5.6 Hz, 2H), 8.00 (d, J = 14.3 Hz, 4H), 7.78 (s, 1H), 7.67-7.60 (m, 2H), 7.54 (d, J = 8.0 Hz, 1H), 7.43 (t, J = 7.7 Hz, 1H), 7.38 (d, J = 8.3 Hz, 1H), 7.29 (t, J = 7.4 Hz, 1H), 6.55 (d, J = 14.2 Hz, 1H), 6.47 (d, J = 14.0 Hz, 1H), 4.35 (d, J = 43.6 Hz, 9H), 3.64 (t, J = 6.1 Hz, 10H), 3.25 (d, J = 8.1 Hz, 3H), 2.85 (t, J = 7.7 Hz, 2H), 2.69 (s, 4H), 2.64-2.58 (m, 5H), 2.39 (p, J = 1.9 Hz, 1H), 2.07-1.99 (m, 4H), 1.81 (s, 2H), 1.70 (d, J = 15.0 Hz, 13H), 1.24 (s, 1H), 0.01 (s, 1H).

### Comparative example 1

This example provides a heptamethine cyanine near-infrared fluorescent dye, which is NY-ICG-04:

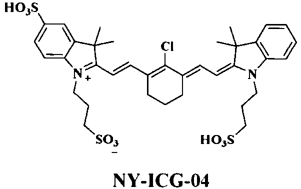

### Comparative example 2

This example provides a heptamethine cyanine near-infrared fluorescent dye, which is

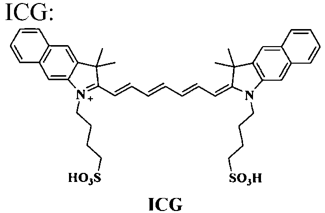

### Comparative example 3

This example provides a heptamethine cyanine near-infrared fluorescent dye, which is YQ-04-SCH₂CH₂CONH(CH₂CH₂O)₂CH₂CH₂NH₂ (from patent CN202110626918.7):

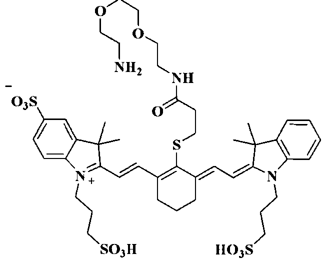

**YQ-04-SCH₂CH₂CONH(CH₂CH₂O)₂CH₂CH₂NH₂**

### Test example 1

### Spectral test

Test samples: heptamethine cyanine near-infrared fluorescent dyes provided in example 1 to example 4.

Test method: each sample as described above was respectively prepared into a 1 nmol aqueous solution; the absorption spectrum of each probe within a range of 500-900 nm was determined using an ultraviolet spectrophotometer (HITACHI, 3J1-0015); and the fluorescence emission spectrum of each probe within a range of 750-850 nm was determined using a microplate reader (Molecule devices, D1524R).

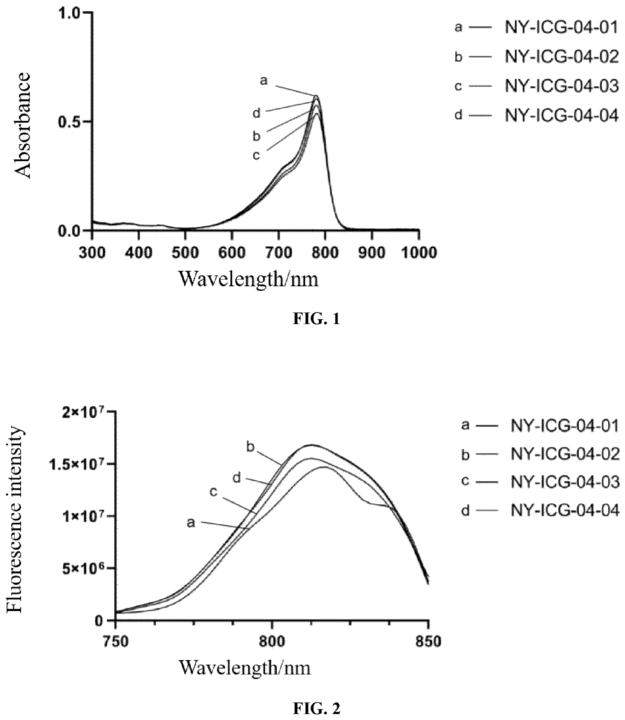

Test results:
The absorption spectrum result of each sample as described above is as shown in FIG. 1, indicating that the maximum adsorption of the heptamethine cyanine near-infrared fluorescent dyes provided in example 1 to example 4 is at about 780 nm.

The emission spectrum result of each sample as described above is as shown in FIG. 2, indicating that the maximum emission spectrum of the heptamethine cyanine near-infrared fluorescent dyes provided in example 1 to example 4 is at about 810 nm.

### Test example 2

### Metabolic distribution imaging in normal mice

Test sample: the heptamethine cyanine near-infrared fluorescent dyes provided in example 1 to example 4, and the heptamethine cyanine near-infrared fluorescent dyes provided in comparative example 1 to comparative example 3.

Test method: in normal ICR mice, each sample as described above (at a dose of 0.5 mg/kg, and glucose injection 100 µL/mouse) was administrated via tail veins, mice were dissected at 4 h, 8 h, 12 h and 24 h to take main organs (heart, liver, spleen, lung, kidney, intestine, stomach, bone, fat, etc.) of mice for fluorescence imaging using a surgical fluorescence imaging system (Nanjing Nuoyuan Medical Equipment Co., Ltd).

### Test results:

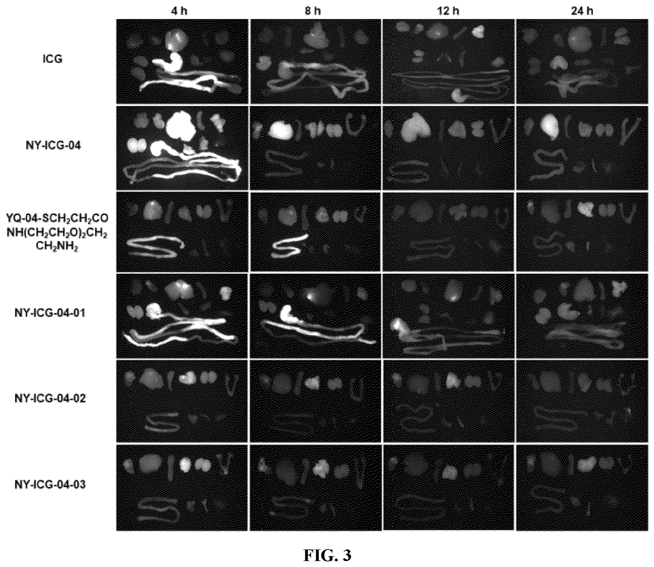

The fluorescence imaging result of each sample as described above is as shown in FIG. 3, indicating that the in-vivo metabolism of the heptamethine cyanine near-infrared fluorescent dyes provided in example 1 to example 3 during the administration of 24 h is basically consistent with that in comparative example 3, with a certain lung fluorescence signal, and the heptamethine cyanine near-infrared fluorescent dyes provided in example 1 to example 3 are basically excreted from liver after 8 h of administration; compared with comparative example 1 (NY-ICG-04), the heptamethine cyanine near-infrared fluorescent dyes provided in example 1 to example 3 have reduced liver accumulation, and can be further developed and applied to detection of liver diseases. Furthermore, the results show that the heptamethine cyanine near-infrared fluorescent dye provided in each example of the present disclosure has an increased in-vivo clearance rate with an increase in the quantity of introduced amino acids; and compared with comparative example 2 (ICG), the in-vivo clearance rate shows a significant increasing trend, and therefore the heptamethine cyanine near-infrared fluorescent dyes provided in example 1 to example 3 have more application advantages.

### Test example 3

### Subcutaneous tumor HepG2 tumor bearing mouse model (human-derived hepatoma cells) live imaging

Test sample: the heptamethine cyanine near-infrared fluorescent dyes provided in example 1 to example 4, and the heptamethine cyanine near-infrared fluorescent dyes provided in comparative example 1 to comparative example 3.

Test method: in a subcutaneous tumor HepG2 tumor bearing mouse model, each sample as described above (at a dose of 0.5 mg/kg, and glucose injection 100 µL/mouse) was administrated via tail veins, indocyanine green (at a dose of 0.5 mg/kg, and glucose injection 100 µL/mouse) was used as control, fluorescence imaging was performed using a surgical fluorescence imaging system (Nanjing Nuoyuan Medical Equipment Co., Ltd, 10B) after administration at 0 h (prior to administration), 6 h, 12 h, 24 h and 48 h in turn.

### Test results:

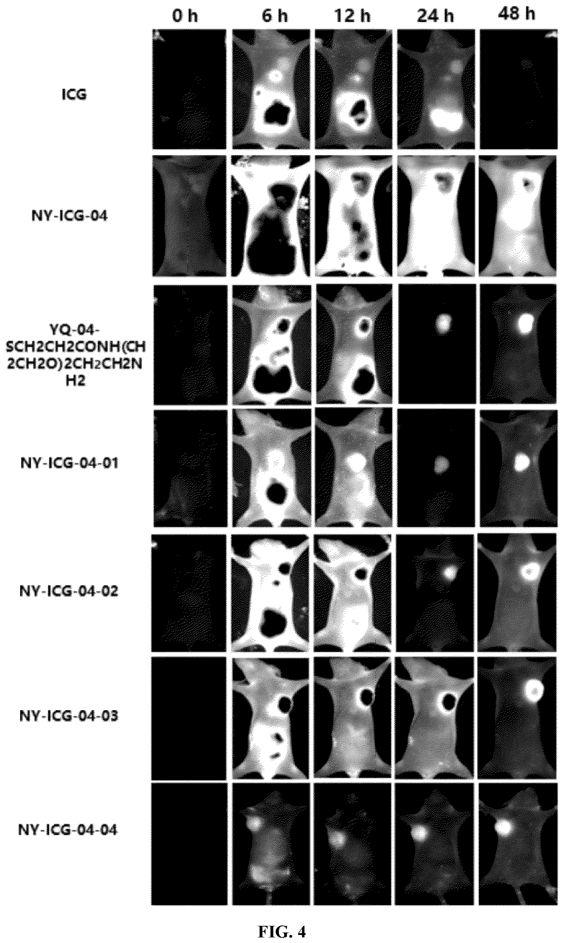

The fluorescence imaging result of each sample as described above is as shown in FIG. 4, indicating that compared with comparative example 2 (ICG), the heptamethine cyanine near-infrared fluorescent dyes provided in examples of the present disclosure have a stronger targeting tumor targeting ability in liver cancer, and longer tumor developing time.

Compared with comparative example 1 (NY-ICG-04), the compound has a basically unchanged tumor targeting ability, and can effectively improve the deficiency of poor liver accumulation and no metabolism in comparative example 1.

Compared with comparative example 3 (YQ-04-SCH₂CH₂CONH(CH₂CH₂O)₂CH₂CH₂NH₂), the compound of the present disclosure has a considerable or stronger tumor targeting ability, a quick excretion rate in normal tissues and a quickly reduced normal tissue background signal, and possesses potential tumor detection ability and clinical application prospects.

### Test example 4

Test sample: the heptamethine cyanine near-infrared fluorescent dye provided in example 3.

Test method: in a colorectal cancer (HCT116) tumor bearing mouse model, a probe NY-ICG-04-03 (at a dose of 0.5 mg/kg, and glucose injection 100 µL/mouse) was administrated via tail veins, fluorescence imaging was performed using a surgical fluorescence imaging system (Nanjing Nuoyuan Medical Equipment Co., Ltd, 10B) after administration at 0 h (prior to administration), 6 h, 12 h, 24 h and 48 h in turn.

### Test results:

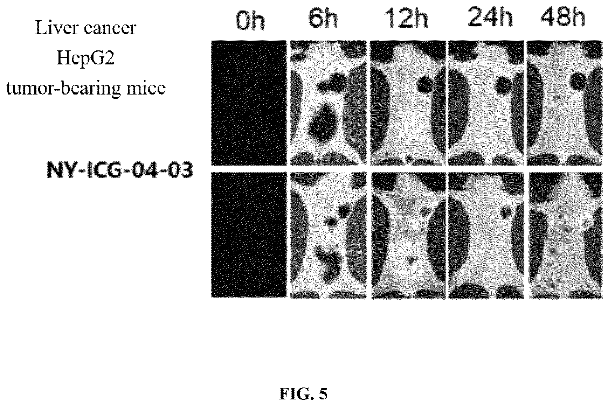

The fluorescence imaging result of the probe NY-ICG-04-03 as described above is as shown in FIG. 5, indicating that the probe NY-ICG-04-03 also has a good tumor targeting ability on colorectal cancer (HCT116) tumor bearing mice, and possesses potential clinical application prospects so as to need further researches and development to be expected for clinical surgery.

Finally, it should be noted that each embodiment as described above is merely for illustrating the technical solution of the present disclosure but not limiting it; although the present disclosure has been described in detail with reference to each embodiment as described above, persons of ordinary skill in the art should understand that they still can make modification to the technical solutions described in the foregoing embodiments, or make equivalent replacement to a part or all of technical features; and these modifications or replacements do not make the natures of corresponding technical solutions depart from the scope of the technical solution of each embodiment of the present disclosure.

## Claims

1. A heptamethine cyanine near-infrared fluorescent dye having a structure represented by formula I: 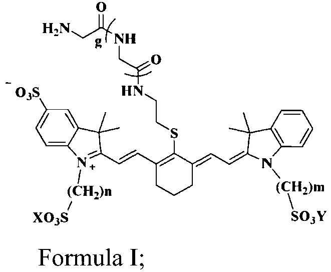 wherein, X or Y is each independently a hydrogen ion or a salifiable positive-valence ion; m or n is each independently 3 or 4; and g is an integer from 0 to 20.

2. The heptamethine cyanine near-infrared fluorescent dye according to claim 1, wherein the salifiable positive-valence ion comprises a positive-valence alkali metal ion and/or NH⁺₄;
wherein, the positive-valence alkali metal ion is Na⁺ and/or K⁺.

3. A preparation method of the heptamethine cyanine near-infrared fluorescent dye according to claim 1 or 2, comprising the following steps:
(1) mixing ((9H-fluoren-9-yl) methoxy) carbonyl triglycine, 2-(triphenylmethylsulfydryl) ethylamine, a condensing agent, an alkali and a reaction solvent, performing a condensation reaction on the above mixed solution and then posttreating the obtained condensation product, so as to obtain an intermediate a;
(2) mixing the intermediate a, trifuloroacetic acid, triisopropylsilane and water for reaction, and then posttreating the obtained reaction product to obtain an intermediate b;
(3) mixing a dye molecule, the intermediate b, the alkali and the reaction solvent for reaction, and then posttreating the obtained reaction product to obtain an intermediate c;
the dye molecule having a structure represented by formula II: 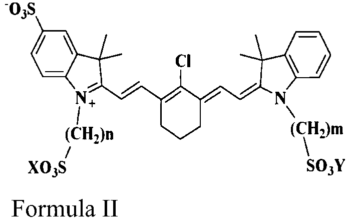
wherein, X or Y is each independently a hydrogen ion or a salifiable positive-valence ion; and m or n is each independently 3 or 4; and
(4) mixing the intermediate c, secondary amine and the reaction solvent for reaction, and then posttreating the obtained reaction product to obtain the heptamethine cyanine near-infrared fluorescent dye.

4. The preparation method of the heptamethine cyanine near-infrared fluorescent dye according to claim 3, wherein in step (1), a molar ratio of the ((9H-fluoren-9-yl) methoxy) carbonyl triglycine to the 2-(triphenylmethylsulfydryl) ethylamine to the condensing agent to the alkali is 1: (0.8-1.2): (1.5-3): (2-4);
and/or, in step (1), a mass ratio of the reaction solvent to the ((9H-fluoren-9-yl) methoxy) carbonyl triglycine is (5-20): 1;
and/or, in step (1), the temperature of the condensation reaction is 10-40°C, and the time of the condensation reaction is 0.5-2 h;
and/or, in step (1), the posttreating specifically comprises the following steps: adding water into a reaction solution obtained after condensation reaction in step (1) to form a suspension; and extracting the suspension with an organic solvent, collecting an organic phase, and then drying and concentrating the collected organic phase to obtain the intermediate a.

5. The preparation method of the heptamethine cyanine near-infrared fluorescent dye according to claim 3 or 4, wherein in step (2), a volume ratio of the trifluoroacetic acid to the triisopropylsilane to the water is (94-96): (2-3): (2-3);
and/or, in step (2), a ratio of a total volume of a mixed solution of the trifluoroacetic acid, the triisopropylsilane and the water to a volume of the organic solvent for extraction is 1: (0.8-1.2);
and/or, in step (2), the temperature of the reaction is 10-40°C, and the time of the reaction is 0.5-2 h;
and/or, in step (2), the posttreating specifically comprises the following steps: the pH of a reaction solution obtained after reaction in step (2) is adjusted to be neutral, and then performing filtration, concentration and column chromatography on the solution after pH adjustment to obtain the intermediate b.

6. The preparation method of the heptamethine cyanine near-infrared fluorescent dye according to claim 3, wherein in step (3), a molar ratio of the dye molecule to the intermediate b to the alkali is 1: (0.8-1.2): (1.5-4);
and/or, in step (3), a mass ratio of the reaction solvent to the intermediate b is (5-20):1;
and/or, in step (3), the temperature of the reaction is 10-40°C, and the time of the reaction is 0.5-2 h;
and/or, in step (3), the posttreating specifically comprises the following steps: adding the organic solvent into the reaction solution obtained after reaction in step (3), and centrifuging and filtering the above mixed solution to obtain the intermediate c; wherein, the organic solvent is ethyl acetate and/or methyl tert-butyl ether.

7. The preparation method of the heptamethine cyanine near-infrared fluorescent dye according to claim 6, wherein in step (4), a molar ratio of the intermediate c to the secondary amine is 1: (1-2);
and/or, in step (4), a mass ratio of the reaction solvent to the intermediate c is (5-20): 1;
and/or, in step (4), the temperature of the reaction is 10-40°C, and the time of the reaction is 2-6 h;
and/or, in step (4), the posttreating specifically comprises the following steps: adding the organic solvent into the reaction solution obtained after reaction in step (4) and filtering the above mixed solution to obtain a crude product, and then purifying the crude product via liquid chromatography to obtain the heptamethine cyanine near-infrared fluorescent dye; wherein the organic solvent is ethyl acetate and/or methyl tert-butyl ether;
and/or, the heptamethine cyanine near-infrared fluorescent dye obtained in step (4) has the structure represented by formula I, and g is 2.

8. The preparation method of the heptamethine cyanine near-infrared fluorescent dye according to claim 7, further comprising the following steps:
(5) mixing the heptamethine cyanine near-infrared fluorescent dye obtained in step (4), a raw material 2, the condensing agent, the alkali and the reaction solvent, performing a condensation reaction on the above materials and then posttreating the condensation product to obtain an intermediate e; wherein the raw material 2 is selected from ((9H-fluoren-9-yl) methoxy) carbonyl triglycine or BOC-glycine 3-COOH; and
(6) performing a deprotection reaction on the intermediate e and then posttreating the obtained reaction product to obtain the heptamethine cyanine near-infrared fluorescent dye;
wherein, the heptamethine cyanine near-infrared fluorescent dye obtained in step (6) has the structure represented by formula I, and g is 5;
and/or, step (5) and step (6) are repeated until the heptamethine cyanine near-infrared fluorescent dye having the structure represented by formula I, in which g is more than 6, is obtained.

9. Use of the heptamethine cyanine near-infrared fluorescent dye according to claim 1 or 2 in preparation of a fluorescent contrast agent.

10. Use of the heptamethine cyanine near-infrared fluorescent dye according to claim 1 or 2 in preparation of a drug for tumor diagnosis.
